# EUROPEAN PATENT SPECIFICATION

(11) **EP 3 318 871 B1**
(45) Date of publication and mention of the grant of the patent: **13.03.2024**
(21) Application number: 16848092.9
(22) Date of filing: 20.09.2016
(51) Int. Cl.: G01N 33/00, G01N 15/06, G05B 19/02, G01N 15/00

(54) **INTERNET OF VEHICLES-BASED AIR QUALITY DETECTING SYSTEM**
FAHRZEUGINTERNETBASIERTES SYSTEM ZUR ERFASSUNG DER LUFTQUALITÄT
SYSTÈME DE DÉTECTION DE LA QUALITÉ DE L'AIR BASÉ SUR L'INTERNET DES VÉHICULES

(30) Priority: 24.09.2015 CN 201510617066
(43) Date of publication of application: 09.05.2018
(73) Proprietor: Zhejiang Geely Holding Group Co., Ltd., Hangzhou, Zhejiang 310051 (CN); Zhejiang Geely Automobile Research Institute Co., Ltd., Taizhou, Zhejiang 317000 (CN)
(72) Inventor: LI, Shufu, Hangzhou Zhejiang 310051 (CN)
(74) Representative: Osterhoff, Utz
(86) International application number: PCT/CN2016/099463
(87) International publication number: WO 2017/050211

(56) References cited:
- EP-A1- 2 199 790
- CN-A- 103 267 716
- CN-A- 103 913 405
- CN-A- 103 969 701
- CN-A- 104 033 999
- CN-A- 104 502 534
- CN-A- 105 068 472
- CN-A- 105 136 993
- CN-U- 202 794 147
- CN-U- 202 869 925
- CN-U- 205 067 451
- JP-A- 2000 193 651
- US-A1- 2008 024 323
- FIRCULESCU ADRIAN-COSMIN ET AL: "Low-Cost Air Quality System for Urban Area Monitoring", 2015 20TH INTERNATIONAL CONFERENCE ON CONTROL SYSTEMS AND COMPUTER SCIENCE, IEEE, 27 May 2015 (2015-05-27), pages 240-247, XP033187947, DOI: 10.1109/CSCS.2015.57 [retrieved on 2015-07-27]
- STEFANIE UIBEL ET AL: "Mobile air quality studies (MAQS) in inner cities: particulate matter PM10 levels related to different vehicle driving modes and integration of data into a geographical information program", JOURNAL OF OCCUPATIONAL MEDICINE AND TOXICOLOGY, BIOMED CENTRAL LTD, LO, vol. 7, no. 1, 2 October 2012 (2012-10-02) , page 20, XP021137250, ISSN: 1745-6673, DOI: 10.1186/1745-6673-7-20
- SHU-CHIUNG HU ET AL: "Measuring air quality in city areas by vehicular wireless sensor networks", JOURNAL OF SYSTEMS & SOFTWARE, ELSEVIER NORTH HOLLAND, NEW YORK, NY, US, vol. 84, no. 11, 16 June 2011 (2011-06-16) , pages 2005-2012, XP028388686, ISSN: 0164-1212, DOI: 10.1016/J.JSS.2011.06.043 [retrieved on 2011-06-23]
- Srinivas Devarakonda ET AL: "Real-time air quality monitoring through mobile sensing in metropolitan areas", Proceedings of the 2nd ACM SIGKDD International Workshop on Urban Computing, UrbComp '13, 1 January 2013 (2013-01-01), page 1, XP055601162, New York, New York, USA DOI: 10.1145/2505821.2505834 ISBN: 978-1-4503-2331-4

## Description

### Technical Field

The present invention relates to the field of air quality detection, and in particular to an air quality detection system based on the Internet of Vehicles.

### Background of the Invention

Fog and haze weather is a state of atmospheric pollution, and the expression of fog and haze is a general expression for the content of various suspended particulate matters in the haze exceeding the standards. In short, the fine drops of water in air is fog, which belongs to a liquid; the haze is a solid, is composed of tiny dust particles, sulfuric acid, nitric acid, inhalable particulate matter and other particles in air, and can make the air become turbid to reduce the visibility; and the fog and the haze are combined to form fog and haze.

Now most of the domestic large and medium-sized cities are facing the invasion of haze, whereby causing great trouble to the people's production and life, so that the prevention and control of fog and haze become national key issues to be addressed, in which the fog and haze broadcast plays an active guiding role on the prevention and control of fog and haze. At present, the air pollution index used in the fog and haze broadcast is obtained directly from the air quality detection data of fixed monitoring points, or the collected fixed point detection data are calculated by a mathematical model and converted into the overall air pollution index of the local city, which is thereby used to guide people's production and life.

US2008024323A1 provides systems and methods for gathering and disseminating travel conditions. These conditions may include traffic congestion and air quality data such as CO, NOx, and Ozone (O₃) concentration levels, or meteorological conditions. In some embodiments, fleet vehicles already operating within a given area are equipped with monitoring devices to collect the desired data. The collected data may be transmitted to a central computer that associates the data with a road map and disseminates the data to the public. Dissemination may occur in a variety of ways including providing the data to radio stations for broadcast, posting the data on a website or pushing the data to navigation systems in vehicles or mobile communications devices such as cell phones and personal data assistants (PDAs).

EP2199790A1 discloses apparatus and systems, preferably using UV spectroscopy, for the dynamic and continuous detection and quantification of a range of chemicals, particularly pollutants, in the environment, and relates to the production of a real-time display or map to display chemical levels in the environment.

CN104502534A teaches a portable car-mounted atmospheric environment real-time monitoring device. The portable car-mounted atmospheric environment real-time monitoring device comprises a gas collection and detection module, a data acquisition control module, a location module, a wireless communication module, a power supply conversion module and a power supply protection module and is used for realizing a real-time in-situ monitoring function for a wide-range atmospheric environment. The gas collection and detection module is used for collecting atmospheric air in real time and for detecting the atmospheric environment; the data acquisition control module is used for acquiring data of a gas sensor module and the positioning module in real time and also used for controlling the work state of the gas collection and detection module; the data acquisition control module is further used for analyzing and packaging the acquired atmospheric environment and location data and displaying the data in real time and also for transmitting the data to a remote management center through the wireless communication module so as to analyze the condition of the atmospheric environment.

CN103969701A discloses a system for realtime monitoring of city full-area air quality. The system comprises an air quality monitoring system and more than one vehicle-mounted intelligent service terminal, each vehicle-mounted intelligent service terminal comprises a GPS (global positioning system) positioning module, an air quality monitoring sensor, an information processing module and a wireless communication module, each information processing module is used for combining longitude and latitude, time and air quality data, each air quality monitoring system comprises a network connection module and a map engine, and each map engine is used for drawing a city full-area air quality realtime monitoring picture according to the air quality data at different positions. It further discloses a realizing method of the system. Realtime monitoring of the city full-area air quality is realized by arranging the air quality monitoring sensors on a lot of vehicles to movably collect the air quality data and utilizing a realtime positioning function of a vehicle-mounted GPS.

Firculescu et. al. disclosed on the 2015 20th international conference on control systems and computer science a low-cost air quality system for urban area monitoring. As traffic related air pollution cannot be traced to a single location, there is the need for a new approach which has the capacity of measuring line-grained air quality that can reflect any detailed variations that may be caused by traffic jams. A solution by implementing a low-cost air quality system using mobile sensing for monitoring the variance of gaseous pollutants in metropolitan areas is disclosed.

Uibel et al. disclosed mobile air quality studies in inner cities within the journal of occupational medicine and toxicology in vol. 7, no. 1 published October 2, 2012. The feasibility of mobile PM analysis in vehicles has been demonstrated.

Shu-Chiung Hu et al. teaches measuring air quality in city areas by vehicular wireless sensor networks within the journal of systems and software vol. 84, no. 11 published on June 16, 2011. A micro-climate monitoring scenario usually requires deploying a large number of sensor nodes to capture environmental information. By exploiting vehicular sensor networks it is possible to equip fewer nodes on cars to achieve fine-grained monitoring.

Srinivas Devarakonda et al. disclosed a real-time air quality monitoring through mobile sensing in metropolitan areas within the Proceedings of the 2nd ACM SIGKDD international workshop on Urban computing on January 1, 2013. Traditionally, pollution measurements are performed using expensive equipment at fixed locations or dedicated mobile equipment laboratories. However, it is taught how a vehicular-based mobile approach for measuring fine-grained air quality in real-time is realized.

However, people often feel that the air pollution index in the fog and haze broadcast is deviated from their actual feeling so as to suspect the accuracy of the air quality data.

### Summary of the Invention

An object of the present invention is to, for the purpose of performing air quality detection more accurately to obtain accurate air quality data, provide an air quality detection system based on the Internet of Vehicles.

The inventor has found that there are a number of reasons why people feel that the air quality data are inaccurate, and one of the key reasons is that the distribution of air quality monitoring points is too sparse. For example, it is reported in a newspaper in Beijing in 2015 that there are 35 air quality monitoring points in Beijing, only a few of which being distributed in major urban areas, only a few air quality monitoring points are provided in medium-sized city, and even no air quality monitoring point is provided in small cities. The inventor has also found that, if the air quality monitoring points are substantially increased in the existing manner, the air detection cost will increase significantly, especially when the number of air quality monitoring points is increased to several orders of magnitude of the number of the existing air quality monitoring points, the cost will increase dramatically. Also, the determination and selection of the layouts to be considered for arranging a large number of air quality monitoring points is also a challenge. Therefore, the present invention provides an air quality detection system based on the Internet of Vehicles.

In particular, the present invention provides an internet of vehicles-based air quality detecting system, comprising an air quality detection device as defined by the claims.

The air quality detection system based on the Internet of Vehicles of the present invention uses each of the vehicles accessed to the Internet of Vehicles as an active air quality monitoring point so as to obtain the air quality data outside the vehicle at the current position where each vehicle is located, and can transmit the air quality data to a server as a data processing center through the Internet of Vehicles in time. Since there may be a very large number of vehicles accessed to the Internet of Vehicles, the present invention can obtain air quality data of many orders of magnitude more than those from the existing fixed monitoring points, so that the sample size sampled by the present invention is relatively comprehensive, and thus the present invention can obtain accurate air quality data, and the air quality detection using the system of the present invention is more accurate.

According to the detailed description of the particular embodiments of the present invention below in conjunction with the accompanying drawings, the above-mentioned and other objects, advantages and features of the present invention will be much clearer to a person skilled in the art.

### Brief Description of the Drawings

Some of the particular embodiments of the present invention will be described below in detail in an exemplary but not limiting way with reference to the accompanying drawings. The same reference signs in the figures indicate the same or similar components or parts. A person skilled in the art should understand that these figures are not necessarily drawn to scale. In the accompanying drawings:
Fig. 1 is a schematic logic control diagram of an air quality detection system based on the Internet of Vehicles according to one embodiment of the present invention;
Fig. 2 is a schematic structural diagram of the air quality detection device shown in Fig. 1;
Fig. 3 is a schematic assembly diagram of the air quality monitoring unit shown in Fig. 2 with an outer air inlet pipe and an inner air inlet pipe, this exemplary assembly not being part of the present invention.
Fig. 4 is another schematic logic control diagram of the air quality detection device shown in Fig. 1.

The symbols in the drawings represent the following meanings:
1 Vehicle, 2 Outer air inlet pipe, 3 Inner air inlet pipe, 4 One-way valve,
100 Air quality detection device,
10 Air quality detection unit,
11 Inlet port,
20 Data transmission unit,
30 Position acquisition unit,
40 Time acquisition unit,
200 Server,
300 Air circulation determination unit.

### Detailed Description of the Invention

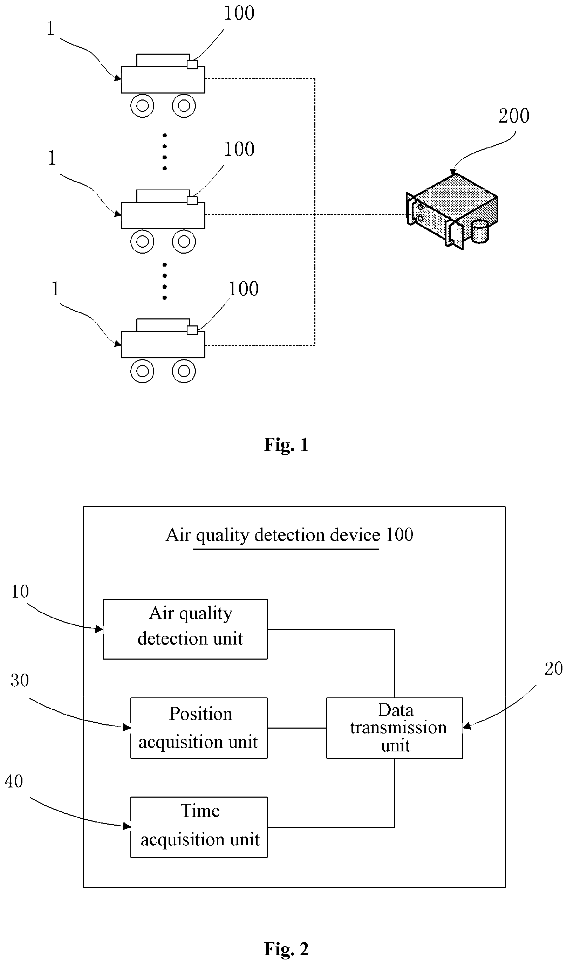

Fig. 1 is a schematic structural diagram of an air quality detection system based on the Internet of Vehicles according to one embodiment of the present invention. In Fig. 1, each vehicle 1 and the server 200 may communicate with each other through the Internet of Vehicles. As shown in Fig. 1, the air quality detection system may comprise an air quality detection device 100 arranged at each vehicle 1 of a plurality of vehicles 1.

In this embodiment, the current position of the vehicle 1 may be any position on the traveling route of the vehicle 1, and may also be a position where the vehicle 1 is parked. In general, the area where the vehicle arrives at is also often an area where the human activities are frequent, so that the air quality data that can be obtained from the positions on the traveling route of the vehicle 1 is more representative of the air quality of the people's actual living area, so that the air quality situations in the fog and haze broadcast are more consistent with the people's actual feelings.

As the annual sale of a large-scale automobile enterprise is generally more than 300 thousand, up to more than 500 thousand, and the vehicles are sold to the provinces, cities and counties all over the country, the air quality detection system based on the Internet of Vehicles of the present invention, therefore, covers the monitoring points much more than the fixed monitoring points arranged by the country, so the detection range can cover most of the country.

For the air quality detection system based on the Internet of Vehicles of the present invention, by arranging an air quality detection device 100 at each vehicle 1 of the plurality of vehicle 1 and by obtaining the air quality data outside the vehicle 1 at the current position where the corresponding vehicle 1 is located by means of the air quality detection device 100, the present invention, therefore, can obtain the air quality data of many orders of magnitude more than those from the existing fixed monitoring points, so that the sample size sampled by the present invention is relatively comprehensive, and thus the present invention can obtain accurate air quality data, so that the air quality detection of the system of the present invention is more accurate and closer to the actual situation, so as to provide more accurate guidance for the people's travel and life.

Fig. 2 is a schematic logic control diagram of the air quality detection device 100 shown in Fig. 1. With reference to Fig. 2, the air quality detection device 100 of each vehicle 1 may comprise an air quality detection unit 10 and a data transmission unit 20. The air quality detection unit 10 is used for acquiring air quality data outside the vehicle 1 at the current position where the corresponding vehicle 1 is located. The data transmission unit 20 is used for transmitting the air quality data to a remote server 200. Specifically, in this embodiment, the data transmission unit 20 may be a vehicle-mounted data transmission system or a part thereof by which the vehicle 1 is accessed to the Internet of Vehicles, so as to take full advantage of the original configuration of the vehicle 1 and reduce the cost. Of course, the data transmission unit 20 may also be an additionally configured unit.

With reference to Fig. 2, in this embodiment, the air quality detection device 100 may further comprise a position acquisition unit 30 for acquiring position data of the current position where the corresponding vehicle 1 is located. The data transmission unit 20 may be configured to transmit the position data of the current position to the server 200 (see Fig. 1) together with the air quality data of the current position. In this way, by simultaneously acquiring the air quality data and the position data of the current position where the vehicle 1 is located and transmitting the data together to the server 200 (see Fig. 1), it is possible to facilitate the drawing of an air quality map on the side of server 200 (see Fig. 1), thus forming a healthy track of life and areas to better guide people's work and travel.

In this embodiment, the position acquisition unit 30 may be a GPS system built in the vehicle 1, so as to take full advantage of the original configuration of the vehicle 1 and reduce the cost. Of course, in other embodiments, the position acquisition unit 30 may also be an additionally provided unit.

It should be understood that, with reference to Fig. 1, when the air quality map is drawn on the side of server 200, it may be necessary to take into consideration of the problem of matching or alignment in time of the air quality data from the vehicles 1. For example, the data from a vehicle 1 may be delayed to reach the server 200 due to network congestion, so that the data received by the server 200 at a certain time may reflect the air quality data in different times.

To this end, with reference to Fig. 2, in this embodiment, the air quality detection device 100 may further comprise a time acquisition unit 40 for acquiring time data of a moment when the corresponding vehicle 1 is located at the current position. The data transmission unit 20 may be configured to transmit the time data of the current position to the server 200 (see Fig. 1) together with the air quality data of the current position. In another embodiment, the data transmission unit 20 may further be configured to transmit the time data of the current position, the air quality data of the current position and the position data of the current position together to the server 200 (see Fig. 1). In this way, on the one hand, it is possible to screen out the obsolete air quality data according to the time data, and on the other hand, when air quality maps are drawn, the air quality maps from the respective vehicles at the same time or in the same period of time (i.e., the data aligned in time) may be used to draw the air quality maps corresponding to this time or this period of time, thereby enabling the air quality map to be time-dependent, and it is possible to observe the change of the air quality maps over time according to the data at different times.

The air quality detection unit 10 may be configured to acquire the air quality data corresponding to a schedule, which comprises one or more discrete scheduled time. The data transmission unit 20 may be configured to transmit only the air quality data corresponding to the schedule to the server 200. A scheduled time in this embodiment refers to any time of the predetermined day, for example, 8:00 a.m., 9:00 a.m., etc. The plurality of discrete scheduled times refer to the times set in accordance with the predetermined rule, for example, the scheduled times every two hours, the times every two hours from 6:00, 8:00, ..... to 24:00. Through the solution described above, it is possible to reduce the amounts of data acquisition and transmitting.

In this embodiment, the plurality of air quality detection units 10 corresponding to the plurality of vehicles 1 (see Fig. 1) use the same schedule. As previously described, when the air quality map is drawn on the side of server 200 (see Fig. 1), it is required that the data is aligned or substantially aligned in time. Through the solution described above, this embodiment can easily realize the alignment in time of the data between the different vehicles, which facilitates the map drawing on the side of server 200 (see Fig. 1).

With reference to Fig. 1, in this embodiment, the system further comprises a server 200 already described above. As previously described, the server 200 may be configured to form an air quality map according to the air quality data from the plurality of vehicles 1.

In one embodiment, the air quality map may be formed by correlating the position data with latitude and longitude coordinates in the map based on the position data and the air quality data at the current position of the vehicle 1 and marking the air quality data corresponding to the position data. By calculating, after further weighted averaging, the overall air quality data in the area and making a contour map of the overall air quality data (which may be the overall PM2.5 value), the general air pollution indexes of the latitudes and longitudes throughout the country can be known as needed. Through the solution described above, this embodiment greatly facilitates the users and provide more accurate guidance for their travel and life.

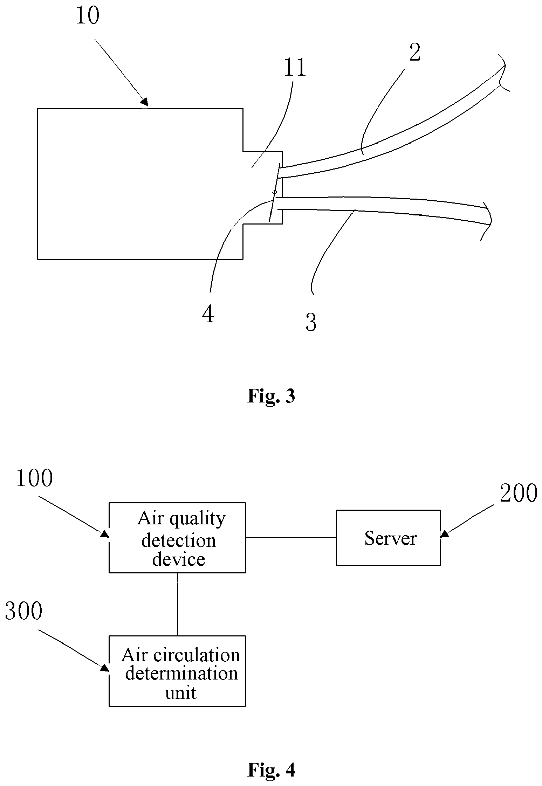

Fig. 3 represents an example not being part of the present invention, and is a schematic assembly diagram of the air quality detection unit 10 shown in Fig. 2 with an outer air inlet pipe 2 and an inner air inlet pipe 3 to represent the arrangement condition of the air quality detection unit 10 at the vehicle 1 (see Fig. 1). As shown in Fig. 3, in this example, the air quality detection unit 10 has an inlet port 11. The inlet port 11 is in communication with the outer air inlet pipe 2 and the inner air inlet pipe 3 separately through a one-way valve 4, an air inlet of the outer air inlet pipe 2 is provided outside the vehicle 1 (see Fig. 1), and an air inlet of the inner air inlet pipe 3 is provided inside the vehicle 1 (see Fig. 1), and by selecting the opening direction of the one-way valve 4, the inlet port 11 is in communication with one of the outer air inlet pipe 2 and the inner air inlet pipe 3 so as to correspondingly acquire the air quality data outside the vehicle 1 (see Fig. 1) or acquire the in-vehicle air quality data inside the vehicle 1 (see Fig. 1). In the specific implementation, the control of the one-way valve 4 can be switched manually by the driver or automatically by the computer periodically, so that it can be automatically displayed on the multimedia screen in the vehicle 1 (see Fig. 1).

With reference to Fig. 3, in this example, the air inlet of the outer air inlet pipe 2 is provided at an intake grille of the vehicle 1 (see Fig. 1). In other examples, the air inlet of the outer air inlet pipe 2 may otherwise be provided in a transition region between a windshield and an engine hood of the vehicle 1 (see Fig. 1), the transition region between the windshield and the engine hood of the vehicle 1 (see Fig. 1) has a certain waterproof and dustproof area, thereby avoiding the possibility of secondary contamination of the air quality detection unit 10, keeping the air inlet of the outer air inlet pipe 2 clean, and helping to ensure the accuracy of the air quality data.

The air quality detection unit 10 can be obtained by modifying the existing vehicle-mounted air quality detector, so that the air quality detection unit 10 can acquire both the air quality data outside the vehicle 1, and also acquire the air quality data inside the vehicle 1, thereby having the advantages of simple operation and convenient use. In other embodiments, the air quality detection unit 10 is dedicated to detecting the air quality data outside the vehicle 1. The air quality detection unit 10 may communicate with the data transmission unit 20 in a wireless or wired manner.

The previously described air quality detection device 100 can be completed as a built-in device at the time of vehicle design and manufacture, or as an additional device installed on an existing vehicle, thereby improving the flexibility of the solution implementation.

Fig. 4 is another schematic logic control diagram of the system shown in Fig. 1. As shown in Fig. 4, the system further comprises an air circulation determination unit 300 for determining whether or not the door or the window of the vehicle 1 (see Fig. 1) has been in an open state for a predetermined time. The air quality detection unit 10 (see Fig. 2) is installed inside the vehicle 1 (see Fig. 1) and acquire the in-vehicle air quality data inside the vehicle 1 (see Fig. 1). The data transmission unit 20 (see Fig. 2), if the result of the determination of the air circulation determination unit 300 is positive, transmits the in-vehicle air quality data as the air quality data outside the vehicle 1 (see Fig. 1) to the server 200.

The air quality detection unit 10 (see Fig. 2) can mainly detect the air quality inside the vehicle 1 (see Fig. 1) and visually display the change of air quality inside the vehicle 1 (see Fig. 1) to the user. The air quality detection unit 10 (see Fig. 2) does not directly detect the air quality outside the vehicle 1. However, the air circulation determination unit 300 makes a determination for the state of the door or the window by receiving a signal of the door switch or the window switch, if the signal displays that the door or the window is opened for a period of time T A A seconds, the in-vehicle air quality data is used as the air quality data outside the vehicle 1 (see Fig. 1).

In this way, the air quality data outside the vehicle is indirectly obtained by means of the air quality detection unit built in a vehicle in the prior art for detecting the air quality data inside the vehicle, so that this solution does not require modification of the existing air quality detection unit, thus eliminating the need for additional costs.

The air quality data in the embodiments described above may be or include PM2.5 data. In this embodiment, the air quality data mainly includes PM2.5 data, but may also include other air quality data, such as nitrogen oxide data and sulfide data, thereby acquiring the concentration of the pollutant outside the vehicle 1 and further feeding back the concentration value of nitrogen oxides, sulfides and other pollutants except for PM2.5 to the users.

In addition, the principle of the air quality detection system of the present invention is not only applicable to the Internet of Vehicles, but also to the detection of the air quality of the user's household purifier and the air quality of the user's mobile phone side, and by installing air quality monitoring devices on the user's household purifier and the user's mobile phone to detect the air quality data of the user's home environment and the user's activity environment, so that the user can clearly know and choose which environment to work, live and travel, thereby improving the user experience.

In addition, it is possible to obtain the difference between the air quality map and the government release information by comparing the air quality map made from the air quality data of the system of the present invention with the PM2.5 concentration released from the national government, so as to correct the air quality map or display the accuracy of the air quality map to the user.

Up to this, a person skilled in the art should recognize that although a plurality of exemplary embodiments of the present invention have been shown and described in detail herein, numerous other variations or modifications meeting the principle of the present invention can be directly determined or derived according to the contents disclosed in the present invention. Therefore, the scope of the present invention is defined by the appended claims.

## Claims

1. An internet of vehicles-based air quality detecting system, comprising an air quality detection device (100) arranged in each of a plurality of vehicles (1), the air quality detection device (100) comprising:
an air quality detection unit (10) for acquiring air quality data outside the vehicle (1) at a current position where the corresponding vehicle (1) is located;
wherein the air quality detection unit (10) is installed inside the vehicle (1) and acquires in-vehicle air quality data inside the vehicle (1); and
a data transmission unit (20) for transmitting the air quality data to a remote server (200),
**characterized in that** the air quality detection device (100) further comprises:
an air circulation determination unit (300) for determining whether or not a door or a window of the vehicle (1) has been in an open state for a predetermined time, and the data transmission unit (20) is configured to, if the result of the determination of the air circulation determination unit (300) is positive, transmit the in-vehicle air quality data as the air quality data outside the vehicle (1) to the server (200).

2. The system according to claim 1, **characterized in that** the air quality detection device (100) further comprises a position acquisition unit (30) for acquiring position data of the current position where the corresponding vehicle (1) is located,
wherein the data transmission unit (20) is configured to transmit the position data of the current position to the server (200) together with the air quality data of the current position.

3. The system according to claim 1 or 2, **characterized in that** the air quality detection device (100) further comprises a time acquisition unit (40) for acquiring time data of a moment when the corresponding vehicle (1) is located at the current position,
wherein the data transmission unit (20) is configured to transmit the time data of the current position to the server (200) together with the air quality data of the current position.

4. The system according to any one of claims 1-3, **characterized in that** the air quality detection unit (10) is configured to acquire the air quality data corresponding to a schedule, which comprises one or more discrete scheduled time; and
the data transmission unit (20) is configured to transmit only the air quality data corresponding to the schedule to the server (200).

5. The system according to claim 4, **characterized in that** the plurality of air quality detection units (10) corresponding to the plurality of vehicles (1) use the same schedule.

6. The system according to any one of claims 1-5, **characterized by** further comprising the server (200), which is configured to form an air quality map according to the air quality data from the plurality of vehicles (1).

7. The system according to any one of claims 1-6, **characterized in that** the air quality data is or comprises PM2.5 data.

## Patentansprüche

1. Internetbasiertes Luftqualitätserfassungssystem von Fahrzeugen, umfassend eine Luftqualitätserfassungsvorrichtung (100), die in jedem einer Vielzahl von Fahrzeugen (1) angeordnet ist, wobei die Luftqualitätserfassungsvorrichtung (100) umfasst:
eine Luftqualitätserfassungseinheit (10) zum Erfassen von Luftqualitätsdaten außerhalb des Fahrzeugs (1) an einer aktuellen Position, an der sich das entsprechende Fahrzeug (1) befindet, wobei die Luftqualitätserfassungseinheit (10) innerhalb des Fahrzeugs (1) installiert ist und fahrzeuginterne Luftqualitätsdaten innerhalb des Fahrzeugs (1) erfasst; und
eine Datenübertragungseinheit (20) zum Übertragen der Luftqualitätsdaten an einen entfernten Server (200),
**dadurch gekennzeichnet, dass** die Luftqualitätserfassungsvorrichtung (100) ferner umfasst:
eine Luftzirkulationsbestimmungseinheit (300) zum Bestimmen, ob eine Tür oder ein Fenster des Fahrzeugs (1) für eine vorbestimmte Zeit in einem offenen Zustand gewesen ist oder nicht,
wobei die Datenübertragungseinheit (20) dazu konfiguriert ist, bei positivem Ergebnis der Bestimmung der Luftzirkulationsbestimmungseinheit (300) die fahrzeuginternen Luftqualitätsdaten als Luftqualitätsdaten außerhalb des Fahrzeugs (1) an den Server (200) zu übertragen.

2. System nach Anspruch 1, **dadurch gekennzeichnet, dass** das Luftqualitätserfassungseinheit (100) weiterhin eine Positionserfassungseinheit (30) zum Erfassen von Positionsdaten der aktuellen Position, an der sich das entsprechende Fahrzeug (1) befindet, umfasst,
wobei die Datenübertragungseinheit (20) dazu konfiguriert ist, die Positionsdaten der aktuellen Position zusammen mit den Luftqualitätsdaten der aktuellen Position an den Server (200) zu übertragen.

3. System nach Anspruch 1 oder 2, **dadurch gekennzeichnet, dass** die Luftqualitätserfassungseinheit (100) weiterhin eine Zeiterfassungseinheit (40) zum Erfassen von Zeitdaten eines Zeitpunkts umfasst, zu dem sich das entsprechende Fahrzeug (1) an der aktuellen Position befindet,
wobei die Datenübertragungseinheit (20) dazu konfiguriert ist, die Zeitdaten der aktuellen Position zusammen mit den Luftqualitätsdaten der aktuellen Position an den Server (200) zu übertragen.

4. System nach einem der Ansprüche 1 bis 3, **dadurch gekennzeichnet, dass** die Luftqualitätserfassungseinheit (10) so konfiguriert ist, dass sie die Luftqualitätsdaten entsprechend einem Zeitplan erfasst, der eine oder mehrere diskrete geplante Zeiten umfasst; und
wobei die Datenübertragungseinheit (20) dazu konfiguriert ist, nur die dem Zeitplan entsprechenden Luftqualitätsdaten an den Server (200) zu übertragen.

5. System nach Anspruch 4, **dadurch gekennzeichnet, dass** die mehreren Luftqualitätserfassungseinheit (10), die der Vielzahl von Fahrzeugen (1) entsprechen, denselben Zeitplan verwenden.

6. System nach einem der Ansprüche 1 bis 5, **dadurch gekennzeichnet, dass** es außerdem den Server (200) umfasst, der so konfiguriert ist, dass er eine Luftqualitätskarte gemäß den Luftqualitätsdaten der Vielzahl von Fahrzeugen (1) erstellt.

7. System nach einem der Ansprüche 1 bis 6, **dadurch gekennzeichnet, dass** die Luftqualitätsdaten PM2,5-Daten sind oder umfassen.

## Revendications

1. Système de détection de la qualité de l'air basé sur l'Internet de véhicules, comprenant un dispositif de détection de la qualité de l'air (100) disposé dans chacun d'une pluralité de véhicules (1), le dispositif de détection de la qualité de l'air (100) comprenant :
une unité de détection de la qualité de l'air (10) pour acquérir des données de la qualité de l'air à l'extérieur du véhicule (1) dans une position actuelle où se trouve le véhicule correspondant (1),
dans laquelle l'unité de détection de la qualité de l'air (10) est installée à l'intérieur du véhicule (1) et acquiert des données de la qualité de l'air à l'intérieur du véhicule (1) ; et
une unité de transmission de données (20) pour transmettre les données de la qualité de l'air vers un serveur distant (200),
**caractérisé en ce que** le dispositif de détection de la qualité de l'air (100) comprend en outre :
une unité de détermination de la circulation de l'air (300) pour déterminer si une porte ou une fenêtre du véhicule (1) a été ouverte pendant une durée prédéterminée,
et l'unité de transmission de données (20) est configurée pour, si le résultat de la détermination effectuée par l'unité de détermination de la circulation de l'air (300) est positif, transmettre les données de la qualité de l'air à l'intérieur du véhicule comme données de la qualité de l'air à l'extérieur du véhicule (1) vers le serveur (200).

2. Système selon la revendication 1, **caractérisé en ce que** le dispositif de détection de la qualité de l'air (100) comprend en outre une unité d'acquisition de position (30) pour acquérir des données de position de la position actuelle où se trouve le véhicule (1) correspondant,
dans lequel l'unité de transmission de données (20) est configurée pour transmettre les données de position de la position actuelle vers le serveur (200) ainsi que les données de la qualité de l'air de la position actuelle.

3. Système selon la revendication 1 ou 2, **caractérisé en ce que** le dispositif de détection de la qualité de l'air (100) comprend en outre une unité d'acquisition de temps (40) pour d'acquérir des données temporelles d'un moment où le véhicule correspondant (1) se trouve à la position actuelle,
dans lequel l'unité de transmission de données (20) est configurée pour transmettre les données temporelles vers le serveur (200) avec les données de la qualité de l'air de la position actuelle.

4. Système selon l'une quelconque des revendications 1 à 3, **caractérisé en ce que** l'unité de détection de la qualité de l'air (10) est configurée pour acquérir les données de la qualité de l'air correspondant à un programme, qui comprend une ou plusieurs heures programmées discrètes ; et
l'unité de transmission de données (20) est configurée pour transmettre uniquement les données de la qualité de l'air correspondant au programme vers le serveur (200).

5. Système selon la revendication 4, **caractérisé en ce que** la pluralité d'unités de détection de la qualité de l'air (10) correspondant à la pluralité de véhicules (1) utilise le même programme.

6. Système selon l'une quelconque des revendications 1 à 5, **caractérisé en ce qu'**il comprend en outre le serveur (200), qui est configuré pour établir une carte de la qualité de l'air en fonction des données de la qualité de l'air de la pluralité de véhicules (1).

7. Système selon l'une quelconque des revendications 1 à 6, **caractérisé en ce que** les données de la qualité de l'air sont ou comprennent des données PM2,5.
